# EUROPÄISCHE PATENTANMELDUNG

(11) **EP 3 459 361 A1**
(43) Veröffentlichungstag der Anmeldung: **27.03.2019**
(21) Anmeldenummer: 17192145.5
(22) Anmeldetag: 20.09.2017
(51) Int. Cl.: A23L 5/20, A23C 11/10, A61K 36/48, A23L 11/00, A23D 7/005

(54) **VERFAHREN ZUR HERSTELLUNG EINES KICHERERBSENQUELLMEHLS**

(71) Anmelder: Agrana Stärke GmbH, 1220 Wien (AT)
(72) Erfinder: NEUWIRTH, Rainer, 3842 Thaya (AT); PRIESNER, Raphael, 3910 Zwettl (AT); WASTYN, Marnik Michel, 2320 Schwechat (AT)
(74) Vertreter: Sonn & Partner Patentanwälte

(57) **Zusammenfassung**

Beschrieben ist ein Verfahren zur Herstellung von Kichererbsenquellmehl zu Verfügung, welches durch die folgenden Schritte gekennzeichnet ist:
- Mahlen von Kichererbsen zu einem Kichererbsenmehl,
- Ansäuern des Kichererbsenmehls auf einen pH-Wert von 4,5 bis 6,0, so dass eine angesäuerte Kichererbsenmehl-Suspension entsteht,
- Erhitzen der angesäuerten Kichererbsenmehl-Suspension,
- Trocknen der erhitzten Suspension zu einem Kichererbsenquellmehl mittels Walzentrocknung.

## Beschreibung

Die Erfindung betrifft die Herstellung von Kichererbsenquellmehl und ein neues Kichererbsenquellmehl mit verbesserten Eigenschaften.

Die Kichererbse (Cicer arietinum), auch Echte Kicher, Römische Kicher, Venuskicher oder Felderbse genannt, ist eine Pflanzenart aus der Gattung Kichererbsen (Cicer) in der Unterfamilie Schmetterlingsblütler (Faboideae) innerhalb der Familie der Hülsenfrüchtler (Fabaceae). Mit der Erbse (Pisum sativum) ist sie nicht näher verwandt. Sie ist eine alte Nutzpflanze.

Die Kichererbse wird im Wesentlichen zur Ernährung des Menschen angebaut. Hauptanbaugebiete der Kichererbse sind heute die Türkei, Nordafrika, Mexiko, Afghanistan, Indien, Pakistan und Spanien. In Mexiko und Indien sind Kichererbsen nach wie vor ein wichtiges Grundnahrungsmittel. Die Produktion weltweit liegt gegenwärtig bei über 13 Mio. Tonnen/Jahr, davon werden fast 9 Mio. Tonnen in Indien produziert.

Kichererbsen enthalten rund 20 % Eiweiß mit einem relativ hohen Anteil der essentiellen Aminosäuren Lysin (10 %) und Threonin (5 %), 40 % Kohlenhydrate, etwa 12 % Ballaststoffe, Vitamin B1, B6 und Folsäure. Der Mineralstoffgehalt an Magnesium beträgt 0,13 %, an Eisen 60 ppm. Getrocknete Kichererbsen haben einen physiologischen Brennwert von ca. 1.152 kJ/100 g (275 kcal/100 g). Verzehrfertige Kichererbsen haben wegen ihres hohen Wassergehalts einen erheblich geringeren Brennwert von etwa 334 kJ/100 g (80 kcal/100 g). Wie alle Bohnenarten verwendet die Kichererbse unter anderem auch Raffinose als Speicherkohlenhydrat, wenngleich nur in relativ geringen Mengen von 290 mg pro 100 g Trockengewicht. Dieser Dreifach-Zucker kann vom Menschen nicht unmittelbar verstoffwechselt werden und gelangt so in den Dickdarm, wo er unter Einfluss der Darmbakterien unter Gasbildung abgebaut wird. Raffinose ist als Ballaststoff anzusehen.

Üblicherweise werden Kichererbsen als Ganzes gekocht, dann püriert und beispielshaft zu Hummus oder Falafel gleich verarbeitet/konsumiert, oder als Frischprodukt angeboten. Diese Produkte haben aber eine eingeschränkte Lagerfähigkeit und müssen gekühlt aufbewahrt werden.

Neben der Direkt-Verwertung in verschiedenen gekochten oder getrockneten Formen ist die (Zwischen-) Verarbeitung zu Kichererbsenquellmehl ein auch großtechnisch genutztes Verfahren, mit welchem dieser Rohstoff transportierbar und lagerfähig gemacht werden kann.

Ein besonderes Problem bei der Verarbeitung von Hülsenfrüchten, insbesondere auch bei Kichererbsen, ist aber der oft als störend empfundene charakteristische "Bohnen-Geschmack" des Mehls und der daraus hergestellten Speisen. Daher wurde der Herstellung von Zitronensäure- und Bicarbonat-behandelten Kichererbsen vermehrt Aufmerksamkeit geschenkt (z.B. Bencini, J Food Sci. 51 (1986), 1518-1525).

Vorgekochte Weisse-Bohnen- und Soja-Mehle mit mildem Bohnen-geschmack wurde durch Vermischen der Mehle mit angesäuertem Wasser, Kochen und Neutralisierung der sauren Suspension und anschließender Walzentrocknung erhalten (Kon et al., J. Food Sci. 35 (1970), 343-345; für weitere Hülsenfrüchte: Kon et al., J. Food Sci. 39 (1974), 897-899). Hierbei wurde festgestellt, dass sich nur bei einem pH von unter 3,85 die erwünschte Geschmacksverbesserung einstellte. Bei diesem pH wurde allerdings die Protein-Extraktion als unzureichend angesehen; erst bei einem pH von 2 wurden sowohl Geschmack als auch die Protein-Extraktion als zufriedenstellend angesehen (Kon et al., 1970). Bei Ansäuerung von Kuhbohnen konnte eine geschmackliche Verbesserung bei Inkubation der ganzen Bohnen bei pH 2 und 6 festgestellt werden, nicht allerdings bei pH 4 (Okaka et al., J. Food Sci. 44 (1979), 1235-1240).

Bei der Anwendung dieser Ansäuerungs-Strategie auf Kichererbsenmehl wurde festgestellt, dass bei pH-Werten von 3,4 und 3.0 die erhaltenen Suspensionen keinen Bohnengeschmack mehr aufwiesen. Bei höheren pH-Werten (konkret getestet wurden Suspensionen bei pH 6,5 und 4,1) konnte dieser Effekt nicht mehr festgestellt (Bencini, 1986) werden.

In dem von Bencini optimierten Verfahren wurden daher die gemahlenen Kichererbsen in 0,01 %-iger Zitronensäure aufgeschlämmt, wodurch eine Suspension mit einem pH von 3,4 erhalten wurde. Diese wurde für 5 min gekocht und dann mit NaHCO₃ auf pH 7 neutralisiert, worauf dann für weitere 45 min gekocht wurde (um das Mehl aufzuschließen; Bencini, 1986; "treatment "(T-2)").

Trotzdem gibt es nach wie vor Probleme bei den funktionalen und sensorischen Eigenschaften derartiger Mehle, die deren Weiterverarbeitung erschweren bzw. deren Akzeptanz beim Konsumenten beeinträchtigen (vgl. z.B. Rackis et al. (J. Am. Oil Chem. Soc. 56 (1979), 262-271), GB 1 385 303 B, DE 2 303 387 A, US 4,022,919 A, US 4,915,972 A, US 2006/0099325 A1, EP 124 165 A2, US 2008/0145511 A1, WO 92/15697 A1, US 2011/0045128 A1, WO 2008/118129 A1).

Die vorliegende Erfindung stellt sich die prinzipielle Aufgabe, ein lagerfähiges Kichererbsenprodukt zur Verfügung zu stellen, welches nicht gekühlt werden muss und somit in einfacher Weise transportiert und gelagert werden kann. Dabei soll ein gegenüber dem Stand der Technik verbessertes KichererbsenMehl bereitgestellt werden, insbesondere ein trockenes Quellmehl, welches lange lagerfähig ist, und jederzeit und ohne Aufwand mit Wasser und anderen Rezepturbestandteilen zu einem konsumierbaren Kichererbsen-basierten Produkt wie z.B. Hummus angerührt werden kann. Dabei soll auch eine gegenüber mit traditionellen Verfahren hergestellten Mehlen verbesserte Sensorik vorhanden sein (weniger Off-taste; De-Flavouring).

Ein weiteres Problem bei der Herstellung von Kichererbsenmehl-Produkten (wie z.B. Hummus) ist auch die Viskosität des wieder aufgeschlämmten Mehls, die in der Verarbeitung problematisch ist; eine Viskositätsverringerung derartiger Produkte ist daher ebenfalls erwünscht. Eine derartige Viskositätsverringerung ist aber bei herkömmlichen Mehlen nicht durch bloße Wasserzufuhr erreichbar, da die Mehle nur eine begrenzte und oft für die industrielle Weiterverarbeitung zu niedrige Wasseraufnahmekapazität aufweisen.

Daher besteht ein Bedarf an verbesserter Herstellungsverfahren für Kichererbsenquellmehle und generell an der Bereitstellung von sensorisch und funktionell verbesserten Kichererbsenquellmehlen, insbesondere ein Kichererbsenquellmehl, das besonders "mild" ist, d.h. wenig Bohnengeschmack aufweist.

Demgemäß stellt die vorliegende Erfindung ein Verfahren zur Herstellung von Kichererbsenquellmehl zu Verfügung, welches durch die folgenden Schritte gekennzeichnet ist:
- Mahlen von Kichererbsen zu einem Kichererbsenmehl,
- Ansäuern des Kichererbsenmehls auf einen pH-Wert von 4,5 bis 6,0, so dass eine Kichererbsenmehl-Suspension entsteht,
- Erhitzen der angesäuerten Kichererbsenmehl-Suspension,
- Trocknen der erhitzten Suspension zu einem Kichererbsenquellmehl mittels Walzentrocknung.

Überraschender Weise konnte mit dem erfindungsgemäßen Verfahren ein einfaches, jedoch trotzdem effektives Verfahren zur Herstellung von Kichererbsenquellmehl zur Verfügung gestellt werden, bei welchem ein effizientes De-Flavouring durch Ansäuern bei relativ hohem pH-Wert, jedoch unter Vermeidung eines Neutralisierungsschrittes erreicht werden konnte. Da im vorliegenden Verfahren lediglich pH-Werte von 4,5 bis 6,0 angewendet werden (und nicht die im Stand der Technik als erforderlich dargestellten, bedeutend geringeren Werte von 3,0 oder 3,4 (Bencini, 1986), können diese pH-Werte auch durch Säuerungsmittel, wie Zitronensaft (Zitronensaftkonzentrat) erzielt werden, die lebensmitteltechnisch und geschmacklich in Zusammenhang mit Kichererbsen-Produkten besondere Vorteile haben.

Es ist daher ein besonderes Merkmal des erfindungsgemäßen Verfahrens, dass sowohl der Erhitzungs- als auch der Trocknungsschritt bei einem pH der Suspension von 4,5 bis 6,0 erfolgt und nicht - wie im Stand der Technik - bei neutralem pH. Ein Neutralisierungsschritt (z.B. ein Versetzen mit NaHCO₃ u. dgl.) entfällt daher im Rahmen des erfindungsgemäßen Verfahrens. Dies war auch deshalb überraschend, da mit der erfindungsgemäßen Verfahrensführung trotz Weglassen des Neutralisationsschrittes ein effektiver Aufschluss des Mehles erreicht werden konnte und gleichzeitig aber auch eine effektive Verringerung störender Geschmackskomponenten, trotz relativ hohem pH im Zuge der vorangehenden Ansäuerung.

Die Kichererbsenmehl-Suspension ist eine Aufschlämmung (engl. "Slurry"), also ein heterogenes Stoffgemisch aus einer Flüssigkeit, hier Wasser, und darin fein verteilten Festkörpern bzw. Partikeln (hier Kichererbsenmehl-Partikeln), die in der Flüssigkeit mit einem Rührer oder Dissolver, aufgeschlämmt werden.

Das Erhitzen dient auch im erfindungsgemäßen Verfahren zum (Stärke-)Aufschluss des (Ausgangs-)Mehls; es zeigte sich, dass dieser Schritt trotz des vergleichsweise niedrigen pH-Wertes gegenüber Kochschritten im Stand der Technik trotzdem effizient zum Aufschluss eignet (und der vergleichsweise hohe pH von 4,5 bis 6,0 bei der Ansäuerung trotzdem zu einem guten De-Flavouring führt). Es zeigte sich auch, dass die Durchführung des Trocknungs-Schrittes bei niedrigem pH zu Vorteilen beim De-Flavouring und zur Herstellung eines geschmacklich besonders vorteilhaften Produktes führt, insbesondere wenn mit Zitronensaft bzw. Zitronensaftkonzentrat als Säuerungsmittel gearbeitet wird.

Besonders vorteilhaft für das erfindungsgemäße Verfahren ist ein Ansäuern auf einen pH-Wert von 4,5 bis 5,5, vorzugsweise 4,4 bis 5,5, insbesondere 5,0 bis 5,5, erfolgt. Diese pH-Werte können somit im großtechnischen Maßstab mit den erfindungsgemäß besonders bevorzugten Säuerungsmittel Zitronensäure, Phosphorsäure, Milchsäure und sauren Fruchtsäften erreicht werden. Die Verwendung von (mit HCl) angesäuertem Wasser (Bencini, 1986; Kon et al., 1970, etc.) ist daher nicht erforderlich. Insbesondere können die geschmacklich besonders bevorzugten Fruchtsäfte, Zitronen- oder Limettensaft, vorteilhafterweise, jeweils als Konzentrate, erfindungsgemäß eingesetzt werden, um die pH-Werte einzustellen. In einer besonders bevorzugten Variante des erfindungsgemäßen Verfahrens wird ein aus kontrolliert biologischer Landwirtschaft erhaltenes Zitronensaftkonzentrat als Säuerungsmittel verwendet. Die Bio-Kontrolle muss dabei natürlich der EU Bio Verordnung Nr. 834/2007 über die ökologische/biologische Produktion und die Kennzeichnung von ökologischen/biologischen Erzeugnissen entsprechen. Typischer Weise sind derartige Zitronensaft-Konzentrate sortenrein und haben pH-Werte von 1,6 bis 2,4. Lösliche Feststoffe derartiger Zitronensaft-Konzentrate können im Bereich von 43-51° Brix, die Dichte bei 1,21 bis 1,27 g/ml liegen; die Konzentrate können von 31,0 bis 35,5 Gew.-% Gesamtsäure als Zitronensäure (pH 8,1) enthalten (standardmäßig gemessen bei 20°C). Vorzugsweise erfolgt das Erhitzen (zur Verkleisterung bzw. Stärke-Aufschluss, also der Überführung in einen kalt-wasser löslichen bzw. kalt-quellenden Zustand) während eines Zeitraums von 5 min bis 60 min, vorzugsweise von 5 bis 40 min, insbesondere von 5 bis 20 min. Dabei richtet sich aber die Zeitdauer stets nach der Effizienz im Stärke-Aufschluss. Dabei hat es sich als zweckmäßig herausgestellt, die erhaltene Suspension, vorzugsweise unter Rühren, auf einen Zieltemperaturbereich von über 70°C, insbesondere von über 75°C, zu erhitzen und dann für zumindest 5 min, insbesondere für mindestens 10 min, auf dieser Temperatur zu halten (vorzugsweise unter fortgesetztem Rühren). Der Aufschluss soll solange durchgeführt werden, bis ein weitgehend vollständiger Aufschluss erhalten wird (was im Regelfall bereits nach 10 min erfolgt sein kann). Eine zu geringe Temperatur (weniger als 65°C, bisweilen auch weniger als 70°C können zu einem unvollständigen Aufschluss der Kichererbsenstärke führen, womit die Wasserbindungsfähigkeit verringert und die Viskosität erhöht werden kann. Dies führt zu einer negativen Beeinflussung des Endproduktes (Hummus), da dieses dann weniger cremig/pastös ist. Auf der anderen Seite können zu geringe Temperaturen auch zu einer Verlängerung des für den Aufschluss erforderlichen Erhitzungsschrittes führen.

Gemäß einer bevorzugten Ausführungsform des erfindungsgemäßen Verfahrens erfolgt das Erhitzen bei einer Temperatur von 65 bis 100°C, vorzugsweise von 70 bis 95°C, insbesondere von 75 bis 90°C (bei Normaldruck).

Vorzugsweise erfolgt die Walzentrocknung - je nach Fabrikat der Walzentrocknungsanlage - während eines Zeitraums von 0,10 min bis 1,00 min, vorzugsweise von 0,11 bis 0,50 min, insbesondere von 0,13 bis 0,40 min. Vorteilhafte Walzendrehzahlen liegen 1,0 bis 20,0 U/min, vorzugsweise von 3,0 bis 10,0 U/min, insbesondere von 3,5 bis 7,0 U/min. Bevorzugt angewendete Walzendrucke können von 2,0 bis 15,0 bar, vorzugsweise von 3,0 bis 10 bar, insbesondere von 4,0 bis 6,5 bar, liegen.

Das Ansäuern des Kichererbsenmehls erfolgt vorzugsweise - in Abhängigkeit des zu erzielenden pH-Werts - durch Zugabe des Säuerungsmittels, wobei die angesäuerte Suspension dann 50 bis 80 %, insbesondere 60 bis 70 %, Trockensubstanz enthält.

Das erfindungsgemäß hergestellte Kichererbsenquellmehl ist nicht nur ein "clean label" Produkt, also ein Produkt, welches keine mit "E-Nummern" in Europa zu kennzeichneten Zusatzstoffen enthält, und kann dadurch auch, wenn Rohstoffe aus biologischen Landbau verarbeitet werden, als "bio" oder "biologisch" gekennzeichnet werden, es ist auch sensorisch signifikant verbessert gegenüber den beispielsweise mit Zitronensäure, Zitrat oder Bicarbonat hergestellten Mehlen. Auch die funktionellen Parameter des erfindungsgemäßen Mehls unterscheiden sich überraschender Weise signifikant gegenüber anders hergestellten Kichererbsenmehlen, insbesondere auch gegenüber den mit Zitronensäure hergestellten Mehlen.

Daher betrifft die vorliegende Erfindung auch ein erfindungsgemäß erhältliches Kichererbsenquellmehl. Dieses Quellmehl kann dann (durch Zugabe von Speise-Öl (also alle zum Verzehr geeigneten, lebensmitteltauglichen Öle, insbesondere pflanzliche Öle), Wasser und Gewürzen unmittelbar zu einem verzehrbaren Lebensmittel-Produkt, wie Hummus, verarbeitet werden ("Instant-Hummus"). Besonders bevorzugt kommen dabei diejenigen Öle zum Einsatz, die einen besonders hohen Gehalt an ein- oder mehrfach ungesättigten Fettsäuren enthalten, wie Sonnenblumen-, Raps-, Distel-, Soja-, Maiskeim-, Erdnuss- und Oliven-Öl (oder Mischungen davon). Die zugesetzten Öle sollten vorzugsweise ebenfalls aus kontrollierter ökologischer/biologischer Produktion stammen und der EU Bio Verordnung Nr. 834/2007 entsprechen.

Das erfindungsgemäße Mehl zeichnet sich durch verbesserte sensorische und funktionelle Merkmale aus (s. oben), die unerwarteter Weise selbst gegenüber dem gemäß Bencini hergestellten Produkt vorliegen. Insbesondere weist das erfindungsgemäße Mehl einen deutlich verringerten Gehalt an Hexanol (genauer: n-Hexanol) auf, einer maßgeblich für den Bohnen-Geschmack verantwortlichen Komponente (Matoba et al., J. Agric. Food Chem. 33 (1985), 852; Samoto et al., Biosci. Biotechnol. Biochem. 62 (1998), 935). So liegt der Hexanol-Wert im erfindungsgemäßen Mehl um mehr als das 10-fache unter dem Hexanol-Gehalt eines üblichen Kichererbsen Ausgangsmehls; es zeigte sich auch, dass der Ansäuerungs-Schritt zu einer Verringerung des Hexanol-Gehaltes von über 50 % führt, so dass das fertige Quellmehl erfindungsgemäß nur mehr einen (geschmacklich unbedeutenden) Maximalwert von 50 ppm Hexanol erreicht, bevorzugter Weise jedoch deutlich darunter liegt.

Eine besonders verbesserte Eigenschaft des erfindungsgemäßen Mehls ist, dass sich daraus - nach Wiedereinweichung - ein Endprodukt (z.B. ein Hummus-Produkt) mit verringerter Viskosität bereitstellen lässt. Es zeigte sich nämlich, dass das erfindungsgemäße Mehl eine höhere Wasseraufnahmekapazität aufweist, als vergleichbare Mehle, insbesondere als das mit Neutralisieren vor dem Erhitzungsschritt hergestellte Mehl.

Ein weiteres Anwendungsgebiet ist die Herstellung eines Falafel Trockenproduktes, wo durch Zumischung des erfindungsgemäßen Mehls zur Hauptkomponente Kichererbsenmehl - nach Wiedereinweichung - die unmittelbare Weiterverarbeitung des Teiges ohne Einhaltung einer mehrminütigen Quellzeit ermöglicht wird.

Insbesondere weisen die erfindungsgemäßen Mehle geschmacklich verbesserte Eigenschaften auf, die sich auch objektiv überprüfen lassen. Beispielsweise haben die erfindungsgemäßen Mehle einen signifikant reduzierten Gehalt an den geschmacklich unerwünschten Stoffen, wie verschiedene, für den Bohnengeschmack verantwortliche Alkohole, Aldehyde oder Ketone, insbesondere Amylalkohol oder Hexanol (s. oben) auf, wohingegen der Gehalt an Hexanal signifikant höher ist als bei vergleichbaren Mehlen. Unter "signifikant" wird gemäß der vorliegenden Erfindung eine Erhöhung bzw. Erniedrigung von zumindest 20%, vorzugsweise von zumindest 50%, insbesondere von zumindest 80%, gegenüber dem Wert verstanden, der mit der Herstellung ohne Zusatz von Zitronensaft bzw. mit Neutralisierungsschritt nach der Ansäuerung erhalten wird.

Insbesondere in sensorischer Hinsicht ist das erfindungsgemäß hergestellte Mehl (und das daraus direkt hergestellte Hummus) besonders ausgezeichnet, wie durch standardisierte Sensorik-Testung mittels professioneller Panels bestätigt werden konnte (siehe Beispielteil) . So wurde das erfindungsgemäße Mehl als "geruchsneutral" und vom Geschmack her "süß" und "mehlartig" bewertet; im Vergleich mit den gemäß Bencini hergestellten Mehlen wurde aber auch besonders die saurere, festere und körnigere Sensorik hervorgehoben. Die Erfindung wird anhand der nachfolgenden Beispiele näher erläutert, ohne jedoch darauf beschränkt zu sein.

### BEISPIELE

Für die nachfolgenden Beispiele wurden im Wesentlichen zwei unterschiedlich hergestellten Kichererbsenquellmehle untersucht: Das erfindungsgemäß hergestellte Mehl ("RW27") und das gemäß Bencini (J. Food Sci. 51 (1986), 1518-1521) hergestellte Mehl ("RW28").

Beim erfindungsgemäßen Produkt (RW27) wurde das Ausgangsmehl mit Wasser zu einer Suspension angerührt, mit Zitronensaftkonzentrat versetzt, im Reaktor verkleistert und auf der Walze getrocknet. Das zweite Produkt (RW28) wurde nach der Rezeptur gemäß Bencini hergestellt, bei dem der Kichererbsen-Slurry mit einer 0,01% Zitronensäurelösung hergestellt wird, der nach der Verkleisterung mit Soda neutralisiert und danach auf der Walze getrocknet wird. Die Produkte wurden beide nach dem Trocknen auf 1,5mm vermahlen.

Die Produkte wurden sowohl sensorisch als auch chemisch analytisch untersucht. Sensorisch zeigen sich deutliche Unterschiede, wobei das RW28 als weicher, wässriger und süßlicher wahrgenommen wird, während sich das RW27 als fester, körniger und sauer darstellt.

Das RW27 hat im Vergleich zum RW28 eine höhere Trockensubstanz, höheres Schüttgewicht und geringere Wasser- und Ölbindung. Dabei ist auch festzuhalten, dass bei der Bestimmung der Wasserbindung der Kuchen des RW27 nach dem Zentrifugieren wesentlich kompakter war als beim RW28.

### 1. Herstellung des Kichererbsenmehls

Das erfindungsgemäße Produkt (mit "RW27" bezeichnet) wurde folgendermaßen hergestellt:
Aus 15 kg Bio-Kichererbsenmehl wird mit 45 kg entmineralisiertem Wasser in einem gerührten 100 l Behälter eine Suspension hergestellt. Nach Zugabe von 0,4 bis 0,5 l Bio-Zitronensaftkonzentrat 30%ig wird der pH bestimmt. Die Suspension wird dann in einen 100 l Stahlreaktor überführt und durch Erhitzen auf über 75°C verkleistert. Dazu wird der Doppelmantel beheizt und Dampf injiziert. Der Kleister wird danach noch 10 min gerührt.

Anschließend wird der Kleister auf einem Walzentrockner aufgetragen, der mit Dampf (5 bar) beheizt wird (der verwendete Dampf zur Erhitzung der Walze hatte etwa 5 bar). Nachdem Dampf mit 5 bar 158°C entspricht, liegt die Oberflächentemperatur der Walze bei etwa 150 bis 155°C. Die Verweilzeit auf der Walze beträgt etwa 20-25 s. Die trockene Schuppe wird mittels Messer abgenommen. Die trockene Schuppe wird dann mittels Schneid-Mühle (1,5 mm Siebeinsatz) vermahlen, um ein einheitliches Pulver zu erhalten.

Das Produkt nach Bencini (RW28) wurde folgendermaßen hergestellt:
Aus 15 kg Bio-Kichererbsenmehl wird mit 45 kg 0,01% Zitronensäurelösung (aus Bio-Zitronensaftkonzentrat 30%ig, pH 3,4) in einem gerührten 100 l Behälter eine Suspension hergestellt. Die Suspension wird dann in einen 100 l Stahlreaktor überführt und durch Erhitzen auf über 75°C verkleistert. Dazu wird der Doppelmantel beheizt und Dampf injiziert. Der Kleister wird danach noch 10 min gerührt. Durch Zugabe einer gesättigten Speisesoda-Lösung (NaHCO₃) wird der Kleister auf pH7 gestellt und weitere 45 min gerührt. Dabei ist zu beachten, dass die 45 min bei Bencini deutlich länger sind als die 10 min oben im erfindungsgemäßen Ansatz, so dass nicht unwahrscheinlich ist, dass im erfindungsgemäßen Verfahren bei 45 min Rührzeit weitere flüchtige Aromastoffe verschwinden.

Anschließend wird der Kleister auf einem Walzentrockner aufgetragen, der mit Dampf (5 bar) beheizt wird. Der Dampfdruck von Bencini war mit 1 bar noch deutlich niedriger. Auch daraus könnte man zusätzlich ableiten, dass im originalen Bencini-Verfahren noch weniger flüchtige Aromastoffe verschwinden. Die trockene Schuppe wird mittels Messer abgenommen. Die trockene Schuppe wird dann mittels Schneid-Mühle (1,5 mm Siebeinsatz) vermahlen, um ein einheitliches Pulver zu erhalten.

Die Produkte wurden folgenden Analysen unterzogen:
1. Trockensubstanzbestimmung mittels Schnelltrockner (Fa. Sartorius, MA35) gemäß ICC-Standard Methode Nr. 110/1.
2. L*a*b*-Farbmessung mittels Farbmessgerät (Fa. Minolta, Chromameter CR 400) gemäß EN ISO 11664-4 durchgeführt.
3. pH-Wert (pH Meter Fa. WTW, pH 3210): Das Kichererbsenmehl wird in Deionat 10%ig in Substanz eingerührt. Danach wird die pH-Elektrode (Kunststoff- Einstabmesskette mit Gelelektrolyt) mindestens bis über beide Elektroden in die am Magnetrührer gerührte Probe getaucht. Sobald sich der Messwert stabilisiert hat, wird der pH-Wert abgelesen.
4. Viskosität nach Brookfield: 110ml Deionat werden in einem 150 ml Becherglas vorgelegt. Darauf werden 16,5 g Kichererbsenmehl (13% in Substanz) eingestreut und unter Rühren mit einem Kunststoff-Stab eine homogene Masse ohne Klumpen hergestellt. Die Masse wird mit der Hand 1 min intensiv weitergerührt. Unmittelbar danach wird die Rotations-Viskosität mittels Brookfield-Viscometer (Typ RVDV-II +PRO, Spindel4, 20 Upm) gemessen. Nach 20 s wird der Wert abgelesen.

Die Ergebnisse sind in der folgenden Tabelle wiedergegeben:

| Probe | | RW27 | RW28 |
|---|---|---|---|
| Bezeichnung | | erfindungsgemäß | Bencini |
| TS | [%] | 95,29 | 92,80 |
| pH (10%) | | 5,2 | 8,3 |
| Viskosität | [mPa.s] | 2300 | 4500 |
| Schüttgewicht | [g/l] | 310 | 264 |
| Wasserbindung | [g/g] | 4,3 | 6,8* |
| Ölbindung | [g/g] | 1,5 | 2,1 |
| Farbe | L | 81,7 | 82,2 |
| | a* | -0,6 | -1,6 |
| | b* | 24,2 | 23,2 |
| | | | *): weniger kompakt |

### 2. Untersuchung der sensorischen Merkmale

Bei der Untersuchung der sensorischen Merkmale wurden die folgenden 2 Rezepturen zur Herstellung eines verzehrfertigen Hummus verwendet (wobei die "Hummus-Mischung" im einen Fall (RW 27) gemäß der vorliegenden Erfindung hergestellt wurde und im zweiten Fall (RW 28) gemäß Bencini (s. oben)):
(a) Hummus RW 27

| | | |
|---|---|---|
| Zutaten | Mengenangaben [Gew-%] | Mengenangaben [g] |
| Hummus Mischung | 16,3 | 40,00 |
| Wasser | 67,2 | 165,32 |
| Olivenöl | 16,1 | 39,70 |
| Salz | 0,4 | 1,00 |
| GESAMT | 100,0 | 245,02 |

(b) Hummus RW 28

| | | |
|---|---|---|
| Zutaten | Mengenangaben [Gew-%] | Mengenangaben [g] |
| Hummus Mischung | 16,3 | 40,00 |
| Wasser | 67,2 | 165,32 |
| Olivenöl | 16,1 | 39,70 |
| Salz | 0,4 | 1,00 |
| GESAMT | 100,0 | 245,02 |

### Durchführung

Zur sensorischen Beurteilung des Produktes, wurde ein Pivot-Test durchgeführt. Dabei wurde die Probe mit einem Standard verglichen und jeweils angegeben, welche Eigenschaften bei der Probe stärker oder schwächer ausgeprägt sind. Es wurden 6 Personen eines geschulten Panels befragt.

Im nachfolgenden Sensorikpanel wurden die beiden Produkte (RW27 entspricht der erfindungsgemäßen Herstellung, RW28 entspricht der Herstellung nach Bencini) hinsichtlich verschiedener Geschmackseindrücke miteinander verglichen (Spalten 1 und 2) sowie im Hinblick auf den Gesamteindruck bewertet (3. und 4. Spalte). Die dabei vergebenen Noten waren 1 bis 3; 1 ist gut, 3 ist schlecht.

| Sensorikpanel (als Hummus) | RW27 | RW28 | RW27 | RW28 |
|---|---|---|---|---|
| | fester | wässriger | 1 | 3 |
| | körniger | weicher | 2 | 2 |
| | grau/grüner | | 1 | 3 |
| | mehliger | | 1 | 2 |
| | nussiger | | 2 | 2 |
| | saurer | süßer | 1 | 3 . |
| | | | 1,3 | 2,5 |

Aus diesen Ergebnissen ergibt sich, dass das erfindungsgemäße Hummus sensorisch wesentliche besser abschneidet als das Hummus, welches nach Bencini hergestellt wurde.

| Sensorik (trocken) | Mehl | Quellmehl | Quellmehl + | |
|---|---|---|---|---|
| Geruch Geschmack | neutral starke grüne, bohnenartige Note | neutral süß, mehlartig | neutral süß, mehlartig | |
| | | | | |
| Analytik (halbquantitativ) | | | | |
| Amylalkohol | *** | * | * | fuselig-grün-artiges Aroma |
| Hexanol | *** | * | * | fuselig-grün-artiges Aroma |
| Indol | *** | - | - | blumig-animalisch-artiges Aroma |
| Hexanal | * | *** | *** | grüner Geruch |

### Quantitative Analyse:

Bei dieser Analyse wurde das Ausgangsmehl mit dem Quellmehl im Hinblick auf den Gehalt an Amylalkohol und Hexanol untersucht (maßgeblich für den "Bohnen-Fehlgeschmack"; vgl. Matoba et al., J. Agric. Food Chem. 33 (1985), 852-855; Samoto et al., Biosci. Biotechnol. Biochem. 62 (1998), 935-940), wobei einmal mit und einmal ohne Zitronensaftkonzentrat (+/- ZSK) gearbeitet wurde.

Bei der dabei angewendeten Methode wurden 15 g des Mehls mit 300 g entionisiertem H₂O (außer bei 167042 und Hummus Premix à 30 g mit 300 g H₂O) gemischt und dann 10 min bei 2000 Umdrehungen zentrifugiert. Anschließend wurde mittels Faltenfilter gefiltert.

50ml des gefilterten Mehlwassers wurden auf Lichrolut EN (200mg, Merck) extrahiert und mit 1,5 µl Dichloromethan:Methanol (95:5) rausgewaschen.

Bei der GCMS (Agilent 7890B/5977B, S/SL Injektor, Autosampler Gerstel Robotic) wurden jeweils 2,5 µl eingespritzt (Splitless; Injektor-Temperatur: 230°) und auf einer Restek Vms 20m x 0.18 mmx 1µm Säule mit Helium als Trägergas chromatographiert (Ofenprogramm: 50°C (3 min), 10°C/min, 250°C (6 min).

Die Kalibration erfolgte mit 0,1 g Hexanol und 0,1 g Amylalkohol (mit Ethanol auf 20 g aufgefüllt (Ausgangslösung)). Standards 1, 2 und 3 waren eine 100fach verdünnte Ausgangslösung (mit EtOH), eine 10fach verdünnte Lösung von Standard 1 und eine 10fach verdünnte Lösung von Standard 2.

In der nachfolgenden Tabelle sind die Wirkung des De-Flavouring hinsichtlich dieser Geschmackskomponenten durch Walzentrocknung und der zusätzliche Effekt durch den Zusatz von Zitronensaftkonzentrat gut ersichtlich:

| Analytik (quantitativ) | Ausgangsmehl 16704 | Quellmehl-ZSK 25840 | Quellmehl+ZSK 25800 |
|---|---|---|---|
| [ppm] | | | |
| Amylalkohol | 150 | 81 | 66 |
| Hexanol | 465 | 73 | 30 |

## Patentansprüche

1. Verfahren zur Herstellung von Kichererbsenquellmehl zu Verfügung, **gekennzeichnet durch** die folgenden Schritte:
- Mahlen von Kichererbsen zu einem Kichererbsenmehl,
- Ansäuern des Kichererbsenmehls auf einen pH-Wert von 4,5 bis 6,0, so dass eine angesäuerte Kichererbsenmehl-Suspension entsteht,
- Erhitzen der angesäuerten Kichererbsenmehl-Suspension,
- Trocknen der erhitzten Suspension zu einem Kichererbsenquellmehl mittels Walzentrocknung.

2. Verfahren nach Anspruch 1, **dadurch gekennzeichnet, dass** das Ansäuern auf einen pH-Wert von 4,5 bis 5,5, vorzugsweise 4,4 bis 5,5, insbesondere 5,0 bis 5,5, erfolgt.

3. Verfahren nach Anspruch 1 oder 2, **dadurch gekennzeichnet, dass** das Ansäuern durch Zugabe eines Säuerungsmittels, ausgewählt aus Zitronensäure, Phosphorsäure, Milchsäure und sauren Fruchtsäften, insbesondere Zitronen- oder Limettensaft, erfolgt.

4. Verfahren nach einem der Ansprüche 1 bis 3, **dadurch gekennzeichnet, dass** das Ansäuern durch Zugabe von Zitronensaft-Konzentrat, insbesondere Zitronensaft-Konzentrat aus biologischer Herstellung, erfolgt.

5. Verfahren nach einem der Ansprüche 1 bis 4, **dadurch gekennzeichnet, dass** das Erhitzen während eines Zeitraums von 5 min bis 60 min, vorzugsweise von 5 bis 40 min, insbesondere von 5 bis 20 min, erfolgt.

6. Verfahren nach einem der Ansprüche 1 bis 5, **dadurch gekennzeichnet, dass** das Erhitzen bei einer Temperatur von 65 bis 100°C, vorzugsweise von 70 bis 95°C, insbesondere von 75 bis 90°C, erfolgt.

7. Verfahren nach einem der Ansprüche 1 bis 6, **dadurch gekennzeichnet, dass** das Erhitzen unter Rühren erfolgt.

8. Verfahren nach einem der Ansprüche 1 bis 7, **dadurch gekennzeichnet, dass** die Walzentrocknung während eines Zeitraums von 0,10 min bis 1,00 min, vorzugsweise von 0,11 bis 0,50 min, insbesondere von 0,13 bis 0,40 min, erfolgt.

9. Verfahren nach einem der Ansprüche 1 bis 8, **dadurch gekennzeichnet, dass** die Walzentrocknung bei einer Walzendrehzahl von 1,0 bis 20,0 U/min, vorzugsweise von 3,0 bis 10,0 U/min, insbesondere von 3,5 bis 7,0 U/min, erfolgt.

10. Verfahren nach einem der Ansprüche 1 bis 9, **dadurch gekennzeichnet, dass** die Walzentrocknung bei einem Walzendruck von 2,0 bis 15,0 bar, vorzugsweise von 3,0 bis 10 bar, insbesondere von 4,0 bis 6,5 bar, erfolgt.

11. Verfahren nach einem der Ansprüche 1 bis 10, **dadurch gekennzeichnet, dass** das Ansäuern durch Zugabe eines Säuerungsmittels erfolgt, wobei die angesäuerte Suspension 50 bis 80 %, insbesondere 60 bis 70 %, Trockensubstanz enthält.

12. Kichererbsenquellmehl, erhältlich nach einem Verfahren gemäß einem der Ansprüche 1 bis 11.

13. Kichererbsenquellmehl nach Anspruch 11 oder 12, **dadurch gekennzeichnet, dass** es einen Gehalt von Hexanol von 50 ppm oder weniger aufweist.

14. Lebensmittel, insbesondere Hummus-Produkt, **dadurch gekennzeichnet, dass** es aus einem Kichererbsenquellmehl nach einem der Ansprüche 11 bis 13 hergestellt ist.

15. Lebensmittel nach Anspruch 14, **dadurch gekennzeichnet, dass** es neben Kichererbsenquellmehl Wasser, Speise-Öl, insbesondere Sonnenblumen-, Raps-, Distel-, Soja-, Maiskeim-, Erdnuss-und/oder Oliven-Öl, und Gewürze enthält.
